(19) **Europäisches Patentamt**

**European Patent Office**

**Office européen des brevets**

(11) **EP 1 547 591 A1**

(12) **EUROPEAN PATENT APPLICATION**
published in accordance with Art. 158(3) EPC

(43) Date of publication:
**29.06.2005 Bulletin 2005/26**

(51) Int Cl.⁷: **A61K 31/352**, A61K 31/375,
A61P 9/00, A61P 43/00,
C07D 311/62, A23L 1/30,
A23L 1/302, A23L 2/00,
A23F 3/14

(21) Application number: **03771411.0**

(22) Date of filing: **29.07.2003**

(86) International application number:
**PCT/JP2003/009617**

(87) International publication number:
**WO 2004/010991 (05.02.2004 Gazette 2004/06)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **29.07.2002 JP 2002219175
02.06.2003 JP 2003156647**

(71) Applicants:
• **Toyo Shinyaku Co., Ltd.**
**Fukuoka-shi, Fukuoka 812-0011 (JP)**
• **SUNTORY LIMITED**
**Osaka-shi, Osaka 530-8203 (JP)**

(72) Inventors:
• **TAKAGAKI, Kinya c/o Toyo Shinyaku Co., Ltd.**
**Fukuoka-shi, Fukuoka 812-0011 (JP)**
• **MITSUI, Takeshi c/o Toyo Shinyaku Co., Ltd.**
**Fukuoka-shi, Fukuoka 812-0011 (JP)**
• **ABE, Keiichi c/o Suntory Research Center**
**Mishima-gun, Osaka 618-0012 (JP)**

(74) Representative: **Schlich, George William**
**Schlich & Co**
**PO Box 48832**
**London WC2H 9WZ (GB)**

(54) **FOOD IMPROVING BLOOD FLOW**

(57) The composition of the present invention contains proanthocyanidins as active components. When the composition further comprises ascorbic acid or a derivative thereof, the fluidity of blood and effect of protecting blood vessels are further improved. This composition may be a food composition or pharmaceutical composition for improving the blood fluidity. Particularly, this composition is useful as a beverage.

EP 1 547 591 A1

**Description**

Technical Field

The present invention relates to a composition for improving blood fluidity that comprises proanthocyanidins as active components. This composition may be a food composition or pharmaceutical composition for improving the blood fluidity. Moreover, the present invention also provides a beverage (beverage composition) that comprises proanthocyanidins and ascorbic acid or a derivative thereof. This beverage is useful as a beverage having blood fluidity improvement properties.

Background Art

In recent years, diseases associated with blood and circulatory system, such as arteriosclerosis and cerebral infarction, and diseases that have a harmful effect on blood circulation, such as hyperlipidemia and diabetes, have been increasing because of changes in life environment including westernization of eating habits, lack of exercise, and excessive stress. These diseases cause a reduced blood flow in microvessels and capillary vessels, and thus it is pointed out that they may have various harmful effects on the body. Moreover, it is also pointed out that blood flow is related to itchy skin, fatigue, and hypertension, for example.

Generally, circulation of blood, that is, blood flow is reduced under the influence of (1) deterioration in the fluidity of blood due to hyperlipidemia and hyperglycemia; (2) reduction in the blood cell fluidity, that is, reduction in the flexibility of erythrocytes and leukocytes or increase in their viscosity; (3) increase in the platelet aggregation ability, and the like. In particular, blood cells, such as erythrocytes and leukocytes, are said to constitute about 40% of blood by volume, and have an effect especially on the fluidity of blood in micro blood vessels. If such a state in which blood flow is reduced continues for a long period of time, then, for example, the flexibility of blood vessels is lost, the flexibility of erythrocytes deteriorates, microvessels become more likely to be clogged with erythrocytes or leukocytes, or a blood clot is more easily formed. All of these phenomena contribute to the development of the circulatory system diseases as described above. In critical cases, the flow of blood may be blocked, resulting in necrosis of tissue in the area of that blockage. Therefore, "blood flow or blood fluidity in the body" is regarded as important in maintaining good health.

Before now, a large number of foods and food components that may improve blood flow have been reported. Examples of familiar foodstuffs include black vinegar and umeboshi (pickled Japanese plum). Moreover, Japanese Laid-Open Patent Publication No. 7-138168 reports that a polar solvent extract of fish bile improves the fluidity of blood. Furthermore, Japanese Laid-Open Patent Publication No. 2002-97143 reports that glucosamine salts or glucosamine derivatives prevent blood clot formation and improve the fluidity of blood. However, the improvement of blood flow is in most cases mainly achieved by an action associated with the fluidity of blood, and no foods or pharmaceutical compositions that contain a component having both the effect of improving blood fluidity and an effect of improving the strength and elasticity of blood vessels, that is, an effect of protecting blood vessels, are provided. Japanese Laid-Open Patent Publication No. 2000-135071 discloses that an extract from wine lees can provide an effect superior to that of a polyphenol-containing extract with respect to blood vessel strengthening properties. However, there is no consideration on the fluidity of blood.

Therefore, there is a demand for a composition for improving blood fluidity that improves blood flow in the body in the true sense, in other words, that improves the fluidity of blood and also has an excellent effect of protecting blood vessels.

Disclosure of Invention

As a result of in-depth research regarding the composition for improving the blood fluidity in the body, the inventors of the present invention found that a composition containing proanthocyanidins as active components has excellent blood fluidity improvement properties and blood vessel protection properties. It seems that this improvement of the blood fluidity is achieved by improvement of the blood cell fluidity such as erythrocytes and leukocytes.

The present invention provides a composition for improving blood fluidity comprising a proanthocyanidin as an active component.

In a preferred embodiment, the composition improves blood cell fluidity.

In a more preferred embodiment, the above-described composition further comprising ascorbic acid or a derivative thereof.

In a further preferred embodiment, the above-described proanthocyanidin comprises at least 20 wt% of OPC (oligomeric proanthocyanidin).

The present invention also relates to a beverage comprising a proanthocyanidin and ascorbic acid or a derivative thereof.

In a preferred embodiment, the proanthocyanidin and the ascorbic acid or derivative thereof are contained at a weight ratio of 1 : 0.1 to 1:500.

In a preferred embodiment, the proanthocyanidin is contained in the beverage in a concentration of 1 mg/L or more.

In a preferred embodiment, the beverage is a tea drink.

In a preferred embodiment, the beverage has blood fluidity improvement properties.

Brief Description of Drawings

Fig. 1 shows fingertip temperatures of subject 1 that were measured immediately after and 3 minutes after the cold water load in the cases where the subject ingested a drink containing a pine bark extract or a drink containing no pine bark extract.

Fig. 2 shows fingertip temperature of subject 2 that were measured immediately after and 3 minutes after the cold water load in the cases where the subject ingested the drink containing a pine bark extract or the drink containing no pine bark extract.

Best Mode for Carrying Out the Invention

Hereinafter, components that are contained in the composition for improving blood fluidity of the present invention will be first described, and then, the composition for improving blood fluidity of the present invention, and a beverage containing proanthocyanidins and ascorbic acid or a derivative thereof will be described. It should be noted that the following description is not limiting the present invention, and it is apparent to those skilled in the art that various alternations can be made within the scope of the spirit of the present invention.

(Proanthocyanidins and raw materials thereof)

In the present specification, proanthocyanidins refer to a group of compounds that are condensation products having flavan-3-ol and/or flavan-3,4-diol as a constituent unit and having a degree of polymerization of 2 or more.

Proanthocyanidins are known to have various activities, a typical example of which is antioxidation properties.

In this specification, among proanthocyanidins, condensation products having flavan-3-ol and/or flavan-3,4-diol as a constituent unit and having a degree of polymerization of 2 to 4 are referred to as oligomeric proanthocyanidins (OPCs). OPCs, which are one type of polyphenol, are potent antioxidants produced by plants, and cannot be produced in the human body.

OPCs are contained concentratedly in portions of plant leaves, bark, or skin or seeds of fruits. More specifically, they are contained in the bark of pine, oak, bayberry, and the like; the fruit or seeds of grape, blueberry, raspberry, cranberry, strawberry, avocado, locust, and cowberry; the hull of barley, wheat, soybean, black soybean, cacao, adzuki bean, and conker; the inner skin of peanuts; and the leaves of ginkgo, for example. Moreover, it is known that OPCs are also contained in cola nuts in West Africa, the roots of Rathania in Peru, and Japanese green tea.

Therefore, for proanthocyanidins, food raw material, such as ground products or extracts from the above-mentioned barks, fruits or seeds that are rich in OPCs, can be used. In particular, it is preferable to use a pine bark extract. Pine bark is especially abundant in OPCs among proanthocyanidins, and thus is preferably used as a raw material of the proanthocyanidins in the present invention.

(Preparation of proanthocyanidins)

Hereinafter, a method for preparing proanthocyanidins will be described taking a pine bark extract that contains OPCs abundantly as an example.

As the pine bark extract, an extract from the bark of plant belonging to Pinales, such as French maritime pine (Pinus martima), Larix leptolepis, Pinus thunbergii, Pinus densiflora, Pinus parviflora, Pinus pentaphylla, Pinus koraiensis, Pinus pumila, Pinus luchuensis, utsukushimatsu (Pinus densiflora form. umbraculifera), Pinus palustris, Pinus bungeana, and Anneda in Quebec, Canada, can be preferably used. Among these, French maritime pine (Pinus martima) bark extract is preferable.

French maritime pine refers to maritime pines that grow in a part of the Atlantic coastal area in southern France. It is known that the bark of this French maritime pine contains proanthocyanidins, organic acids, and other bioactive substances, and proanthocyanidins, which are the main component of French maritime pine, have a potent antioxidation ability of removing active oxygen.

The pine bark extract is obtained by extracting the bark of the above-described pines using water or an organic solvent. When water is used, warm water or hot water can be employed. As the organic solvent that can be employed for extraction, an organic solvent that is acceptable for production of foods or pharmaceuticals can be employed. Examples of such solvent include methanol, ethanol, 1-propanol, 2-propanol, 1-butanol, 2-butanol, acetone, hexane, cyclohexane, propylene glycol, aqueous ethanol, aqueous propylene glycol, methyl ethyl ketone, glycerin, methyl acetate, ethyl acetate, diethyl ether, dichloromethane, edible oils or fats, 1,1,1,2-tetrafluoroethane, and 1,1,2-trichloroethene. These water and organic solvents may be used alone or in combination. In particular, hot water, aqueous ethanol, and aqueous propylene glycol are preferably used.

The method for extracting proanthocyanidins from pine bark is not particularly limited, and heat extraction or supercritical fluid extraction can be employed, for example.

Supercritical fluid extraction is a method for performing extraction using a supercritical fluid. A supercritical fluid is in a state that is above the liquid-vapor critical point in the phase diagram showing critical temperature and critical pressure. Examples of compounds that can be employed as a supercritical fluid include carbon dioxide, ethylene, propane, and nitrous oxide (laughter gas). Carbon dioxide is preferably used.

Supercritical fluid extraction includes an extraction step in which a target component is extracted with a supercritical fluid and a separation step in which the target component is separated from the supercritical fluid. In the separation step, any separation process can be employed, examples of which include a separation based on a change in pressure, a separation based on a change in temperature, and a separation using an adsorbent or absorbent.

Moreover, it is also possible to perform supercritical fluid extraction in which an entrainer is added. In this method, extraction is performed using an extracting fluid obtained by adding, for example, ethanol, propanol, n-hexane, acetone, toluene, or an other aliphatic lower alcohol, aliphatic hydrocarbon, aromatic hydrocarbon, or ketone at about 2 to 20 W/V% to a supercritical fluid, so that the solubility of a target substance to be extracted, such as OPCs and catechins (described later), in the extracting fluid is dramatically increased or the selectivity of separation is enhanced. Thus, a pine bark extract is obtained efficiently.

Since supercritical fluid extraction can be performed at a relatively low temperature, it has the following advantages: it is applicable for extracting substances that deteriorate or decompose at high temperatures; the extracting fluid does not remain; and the extracting fluid can be recovered and recycled, so that a step of removing the extracting fluid and the like, can be omitted, and thus, the process can be simplified.

Furthermore, methods other than these mentioned above can be employed for extraction from pine bark, the examples of which include a batch method using liquid carbon dioxide, a reflux method using liquid carbon dioxide, a reflux method using supercritical carbon dioxide, and the like.

It is also possible to employ a combination of a plurality of extraction processes to perform extraction from pine bark. By combining a plurality of extraction processes, pine bark extracts with various components can be obtained.

The pine bark extract that is used as proanthocyanidins in the composition for improving blood fluidity of the present invention is specifically prepared using the following method. However, this method is merely an example and the present invention is not limited to this method.

First, 1 kg of the bark of French maritime pine is immersed in 3 L of a saturated solution of sodium chloride, and extraction is performed for 30 minutes at 100°C to obtain an extract liquid (extraction step). Then, the extract liquid is filtrated, and the resultant insoluble material is washed with 500 mL of a saturated solution of sodium chloride to obtain a washed liquid (washing step). The extract liquid and the washed liquid are combined to obtain a crude extract of pine bark.

Next, 250 mL of ethyl acetate is added to this crude extract, mixed, and separated to obtain an ethyl acetate layer. This process is repeated five times, and the obtained ethyl acetate layers are combined. The resultant ethyl acetate extract is added directly to 200 g of anhydrous sodium sulfate for dehydration. Then, this ethyl acetate extract is filtrated, and the filtrated extract is concentrated under a reduced pressure to a volume of 1/5 of the original filtrate. The concentrated ethyl acetate extract is poured into 2 L of chloroform and stirred, and the resultant precipitate is recovered by filtration. Subsequently, this precipitate is dissolved in 100 ml of ethyl acetate, and then the resultant solution is added to 1 L of chloroform to form a precipitate. This process is repeated twice and thus, a washing process is accomplished. With this method, for example, about 5 g of pine bark extract containing at least 20 wt% of OPCs that have a degree of polymerization of 2 to 4 and at least 5 wt% of catechins can be obtained.

In the present invention, in view of the safety when used in foods and drugs, it is preferable to use a pine bark extract having a high proanthocyanidin content obtained from a process that comprises the steps of extracting proanthocyanidins from pine bark using ethanol or water, more preferably while heating, and treating the extract with the use of an adsorption resin (e.g., DIAION HP-20, Sephadex-LH20, and chitin) or an ultrafiltration membrane.

(Proanthocyanidins used for the present invention)

In the composition for improving blood fluidity of the present invention, extracts from the above-described raw material plants, which are typically used for proanthocyanidins, can be employed. In particular, pine bark extracts are preferred, because the pine bark extracts contain a large amount of proanthocyanidins that are condensation products having flavan-3-ol and/or flavan-3,4-diol as a constituent unit and having a degree of polymerization of 2 or more. Among these, pine bark extracts that contain a large amount of condensation products having a lower degree of polymerization are preferably used. As such condensation products, condensation products having a degree of polymerization of 2 to 30 (dimer to 30-mer) are preferable, condensation products having a degree of polymerization of 2 to 10 (dimer to decamer) are more preferable, and condensation products having a degree of polymerization of 2 to 4 (dimer to tetramer; i.e., OPCs) are even more preferable.

In the present invention, proanthocyanidins containing at least 20 wt% of OPCs are preferably used. More preferably, the OPC content is at least 30 wt%. As such proanthocyanidins, pine bark extracts are preferably used.

When proanthocyanidins having a high OPC content are used, a better blood fluidity improvement effect can be achieved than in the case where proanthocyanidins having a high degree of polymerization (having a low OPC content) are used.

There is no particular limitation regarding the proanthocyanidin content in the plant (bark) extract, but it is preferable that the proanthocyanidin content in the extract is less than 80 wt%, preferably less than 75 wt%, and more preferably less than 55 wt% because the bioactivity of the proanthocyanidins themselves may be lowered when the proanthocyanidin content in the plant (bark) extract is high.

Since OPCs are antioxidants as described above, they also provide an effect of reducing the possibility of adult diseases, such as cancer and cardiac diseases, an effect of improving allergic diathesis, such as arthritis, atopic dermatitis, and pollenosis, and an effect of inhibiting oxidation and degradation of collagen, and the like.

Furthermore, OPCs improve absorption of vitamin C in the body and the retention of vitamin C in the body dramatically, and synergistically enhance antioxidation effect in the body.

Furthermore, it is known that in addition to the antioxidation ability, OPCs also provide an effect of recovering the strength and elasticity of blood vessels, an effect of decreasing cholesterol and LDL in blood, an effect of decreasing blood pressure with respect to hypertension, an effect of preventing adhesion of cholesterol, an effect of regenerating vitamin E that has been degraded by active oxygen, an effect of serving as an enhancer of vitamin E, and the like.

In particular, by virtue of the antioxidation effect, the effect of decreasing cholesterol in blood, the effect of decreasing blood pressure with respect to hypertension, the effect of recovering the elasticity of blood vessels, and the effect of preventing adhesion of cholesterol, it is possible to protect blood vessels and also improve the fluidity of blood so as to synergistically improve blood flow in the body.

It is known that a reduction in the fluidity of erythrocytes or leukocytes leads to a reduction in blood fluidity especially in microvessels. The reduction in this fluidity is caused by chemical or physical stimulation, such as an increase in the viscosity of blood cells due to stress of oxidation or inflammation, and the like, change in blood pressure, and vascular constriction. Regarding erythrocytes, it has already been found that the fluidity of erythrocytes is reduced when chemical or physical stimulation, that is, an extrinsic signal is transmitted to the inside of erythrocytes to cause a biochemical change. Since OPCs have properties of enhancing the antioxidation effect and enhancing the strength and elasticity of blood vessels as described above, such chemical or physical stimulation to erythrocytes and leukocytes can be reduced by OPCs, and thus the blood cell fluidity is maintained, so that blood fluidity can be improved. Moreover, when the composition for improving blood fluidity of the present invention is ingested, the blood fluidity can be improved by improving the blood cell fluidity without affecting the platelet aggregation ability and platelet count, blood plasma components such as cholesterol and neutral fat, erythrocyte count, leukocyte count, and the like.

(Ascorbic acid and derivatives thereof)

Ascorbic acid or a derivative thereof may be added so that the proanthocyanidins employed in the present invention, in particular, OPCs, can exert their effects more efficiently. When the composition of the present invention is formulated into a beverage, the effects of the proanthocyanidins can be increased even more by making the proanthocyanidins coexist with ascorbic acid or a derivative thereof.

As ascorbic acid or a derivative thereof to be contained in the composition for improving blood fluidity of the present invention, ascorbic acid or derivatives thereof that are used as food additives, such as ascorbyl glycoside, sodium ascorbate, and magnesium ascorbate, can be used. Natural materials that contain ascorbic acid abundantly (e.g., natural materials derived from fruits such as lemon, orange, and acelora, and natural materials derived from vegetables such as broccoli, Brussels sprouts, pimento, Brassica campestris, and cauliflower) also can be used as the ascorbic acid in the present invention.

When the ascorbic acid or derivative thereof is ingested together with the proanthocyanidins (in particular, OPCs), the absorptivity and the persistence of bioactivity of the ascorbic acid are increased. In the present invention, ascorbic acid or a derivative thereof may be added in order to protect blood vessels, particularly in order to enhance the flexibility and strength of blood vessels and decrease cholesterol in blood. In particular, ascorbic acid and derivatives thereof are known to have an ability of promoting synthesis of collagen that is a structural protein not only of blood vessels but also of every tissue, an ability of reducing stresses (in particular, stress by oxidation), an antithrombotic ability, and an ability of increasing immune strength. Therefore, they can provide not only the effects of protecting blood vessels and improving the fluidity of blood but also an effect of improving the entire tissue in the body.

Moreover, when proanthocyanidins are contained in an aqueous solution, for example, when proanthocyanidins are contained in a beverage, it is effective to employ ascorbic acid or a derivative thereof in order to maintain the bioactivity of the proanthocyanidins. Furthermore, the following effects, for example, are expected to be achieved by adding ascorbic acid or a derivative thereof into a beverage: a flavor and a fragrance are provided for the beverage, and coloration of the beverage is prevented, and the components of the beverage are kept stable.

There is no particular limitation regarding the amount of ascorbic acid or derivative thereof, and the proanthocyanidins and ascorbic acid or derivative thereof are contained in the composition for improving blood fluidity of the present invention at a weight ratio of preferably 1 : 0.1 to 1:500, more preferably 1 : 0.2 to 1:200, and even more preferably 1 : 0.2 to 1:150.

(Catechins)

The composition for improving blood fluidity of the present invention may further contain catechins, if necessary. The term "Catechins" is a general term referring to polyhydroxyflavan-3-ols. As the catechins, for example, (+)-catechin, (-)-epicatechin, (+)-gallocatechin, (-)-epigallocatechin, epigallocatechin gallate, and epicatechin gallate are known. Gallocatechin, afzelechin, and 3-galloyl derivatives of (+)-catechin or gallocatechin are isolated from natural products, in addition to (+)-catechin that is called catechin in a narrow sense. Catechins are known to have a cancer inhibiting ability, an arteriosclerosis preventing ability, a lipid metabolism disorder inhibiting ability, a blood pressure elevation inhibiting ability, a platelet aggregation inhibiting ability, an antiallergic ability, an antiviral ability, an antibacterial ability, a dental caries preventing ability, a halitosis preventing ability, an intestinal flora normalization ability, an active oxygen or free radical eliminating ability, an antioxidation effect, and the like. Moreover, catechins are known to have an antidiabetic effect that inhibits an elevation of blood glucose. Catechins alone have poor solubility in water and exhibit low bioactivity, but the solubility is increased and the catechins are activated in the presence of OPCs. In this way, catechins work effectively when ingested together with OPCs.

By containing such catechins, the composition of the present invention can exert a further excellent effect of improving blood fluidity.

Proanthocyanidin contents derived from raw material plants, in particular plant extracts, often contain catechins as well as OPCs. It is preferable that catechins are contained in the above-described raw material plant extracts in a ratio of 5 wt% or more, and preferably 10 wt% or more. Alternatively, it also preferable that a formulation is prepared so that it contains a raw material plant extract containing at least 20 wt% of OPCs and furthermore, contains catechins in a ratio of 5 wt% or more. For example, when the catechin content in a pine bark extract is less than 5 wt%, it is possible to add catechins so that the catechin content becomes at least 5 wt%. It is most preferable to use a pine bark extract containing at least 5 wt% of catechins and at least 20 wt% of OPCs.

(Other components)

The composition for improving blood fluidity of the present invention may further contain other components that are known to improve blood fluidity, if necessary. Examples of such components include, but are not limited to, black vinegar or ume (Japanese plum) flesh and their extracts; sulfur-containing organic compounds contained in onion or garlic or their extracts; tartary buckwheat; chitin and chitosan and derivatives thereof; glucosamine salts and derivatives thereof; hesperidin, quercetine, or rutin, and their derivatives; vitamins such as vitamin B group, vitamin E, and vitamin K; and water-soluble dietary fibers.

In particular, in order to enhance the ability of suppressing blood glucose level, lipid level in blood, and high blood pressure, and to enhance the antithrombotic ability and the ability of decreasing cholesterol in blood, it is preferable to employ the above-described sulfur-containing organic compounds, vitamin K, vitamin E, or chitin, chitosan, or derivatives. In order to enhance the blood vessel protection and the antioxidaion, hesperidin, quercetine, or rutin or their derivatives can be preferably used.

Moreover, the composition for improving blood fluidity of the present invention may contain additives, such as excipients, extenders, binders, thickners, emulsifiers, lubricants, humectants, suspending agents, coloring agents, flavors, and food additives, as appropriate.

Furthermore, nutritions, such as royal jelly, vitamins, proteins, calcium substances such as eggshell calcium, lecithin, chlorella powder, Angelica keiskei powder, and molokheiya powder, also can be added. It is also possible to add stevia powder, ground green tea powder, lemon powder, honey, maltitol, lactose, sugar solutions, seasoning agents, and the like, so as to control taste.

(Composition for improving blood fluidity)

The composition for improving blood fluidity of the present invention contains proanthocyanidins as active components and may contain ascorbic acid or a derivative thereof, catechins, and other components, if necessary. The proanthocyanidin content in the composition is not particularly limited, but the proanthocyanidins are preferably contained in an amount of 0.0001 wt% to 50 wt%, and more preferably 0.005 wt% to 20 wt% in the composition. Moreover, it is preferable that the amount of ascorbic acid or a derivative thereof to be ingested together with the proanthocyanidins is 0.03 g to 1 g. Such a composition can be used for foods or drugs.

The composition for improving blood fluidity of the present invention can be made into various forms by subjecting the above-described components to processing that can be conducted easily by those skilled in the art. For example, the composition may be prepared in the form of tablets or pills by adding an excipient or the like to a pine bark extract containing proanthocyanidins, or it may be prepared in the form of powder or in other forms without being shaped. Examples of other forms include the forms of capsules such as hard capsules and soft capsules, powder, granule, tea bags, candy, liquid, and paste. Among these, a liquid form (e.g., beverage) is preferable.

Regarding the method for ingesting the composition for improving blood fluidity of the present invention, there is no particular limitation. According to the form of the composition or according to the preference, the composition may be eaten or drunk as it is, or may be dissolved in water, hot water, milk, or the like and drunk. Alternatively, a liquid containing the components of the composition obtained by percolation may be drunk.

(Beverage containing proanthocyanidins and ascorbic acid or a derivative thereof)

Among the compositions for improving blood fluidity of the present invention, a proanthocyanidin-containing beverage that contains water, which is regarded as important in improving blood fluidity, is preferable for further enhancing the blood fluidity improving properties. Furthermore, a proanthocyanidin-containing beverage that contains ascorbic acid or a derivative thereof is more preferable. In this case, the amount of water is preferably 100 mL or more.

The proanthocyanidin content in the above-described beverages is not particularly limited, but the proanthocyanidins may be contained in the beverage at a ratio of 1 mg/L or more, preferably 1 mg/L to 20 g/L, and more preferably 2 mg/L to 10 g/L. Furthermore, when ascorbic acid or a derivative thereof is contained, the weight ratio of the proanthocyanidins and the ascorbic acid or derivative thereof may be in the range of 1 : 0.1 to 1 : 500, preferably 1 : 0.2 to 1 : 200, and more preferably 1 : 0.2 to 1 : 150.

In the above-described beverages, it is preferable to use pine bark extracts for the proanthocyanidins. Pine bark extracts have high solubility in polar solvents such as water and ethanol, and provide a low level of bitter taste although they have a high proanthocyanidin content, so that they can be applied for a wide range of beverages.

It should be noted that the proanthocyanidins in a beverage have a characteristic flavor at a concentration higher than 10 mg/L, but at a concentration of 10 mg/L or less, that flavor can be reduced to a level that is acceptable in drinks, and thus the masking is not needed. The proanthocyanidins are contained in a beverage preferably at a concentration of 10 mg/L or less, and more preferably 5 mg/L or less.

It was confirmed that when the proanthocyanidins are contained in a beverage at a high concentration, it is effective to add the proanthocyanidins to a tea, such as green tea, pine needle tea, oolong tea, black tea, barley tea, and other blended teas, in order to mask the unique flavor of proanthocyanidins effectively with a natural material. By the addition of the proanthocyanidins to tea in a ratio of 2 to 200 mg/L, preferably 5 to 200 mg/L, and more preferably 30 to 200 mg/L, the proanthocyanidins can be contained in the tea without degrading the flavor of the tea. In particular, since Japanese teas contain a large amount of catechins, the blood fluidity improvement property is further enhanced. Also, regarding a beverage of 100 mL or more, proanthocyanidins can be contained at a high concentration as is similar to the case mentioned above when a flavor or a natural fruit juice (e.g., lemon juice) having a potent masking effect is contained in that beverage.

For such beverages, for example, fruit juice drinks, carbonated drinks, sports drinks, and the like are preferable as acidic beverages, and tea drinks, coffee drinks, cocoa drinks, soups, and the like are preferable as low acidic beverages, and they are used as health beverages. Among these, tea drinks are more preferable. Tea drinks contain catechins as described above, so that a blood fluidity improvement effect that previous tea drinks have never possessed can be additionally obtained by adding proanthocyanidins or a plant (bark) extract containing proanthocyanidins to tea drinks. The above-described effect in tea drinks is better than the effect in the other drinks described above, and such tea drinks are useful as health beverages.

Although there is no limitation regarding the intake amount of the composition for improving blood fluidity of the present invention and the beverage (beverage composition) containing proanthocyanidins and ascorbic acid or a derivative thereof, it is preferable that the amount of proanthocyanidins in one intake is 0.001 to 0.2 g, preferably 0.002 to 0.15 g, and more preferably 0.002 to 0.08 g, in order to achieve the blood fluidity improvement effect. With such an amount of intake, an excellent blood fluidity improvement effect associated with improvement of the fluidity of blood cells such as erythrocytes and leukocytes, can be achieved. The amount of intake is much less than the intake of conventional compositions for improving blood fluidity. Furthermore, when the pine bark extract is employed for the proanthocyanidins in the composition of the present invention, the above-mentioned effects can be obtained when the daily intake amount of the proanthocyanidins in the pine bark extract is 0.001 g to 0.05 g, more preferably 0.001 g to 0.04 g, even more preferably 0.001 g to 0.025 g, and most preferably 0.008 g to 0.025 g. This amount of the proanthocyanidins corresponds to the amount of pine bark extract of about 0.002 g to 0.2 g, preferably 0.01 g to 0.15 g, and even more preferably 0.04 g to 0.15 g. When ingested as a beverage, it is preferable that pine bark extract is contained in one intake in an amount of 0.002 g to 0.2 g, preferably 0.01 to 0.15 g, and even more preferably 0.04 g to 0.15 g. Although the reason is not clear, among the plant extracts containing proanthocyanidins, pine bark extracts obtained by extraction using water, hot water, or ethanol can provide effects of improving the blood cell fluidity and improving the blood fluidity that are higher than those of other plant extracts, and such extracts can be used preferably.

The composition for improving blood fluidity of the present invention improves the fluidity of blood and the fluidity of blood cells, and furthermore, improves the flexibility and strength of blood vessels. Therefore, the effect of improving blood fluidity in the living body, particularly the effect of improving peripheral blood fluidity can be achieved. Furthermore, improvement of blood fluidity leads to improvement of the health status of the entire body.

Examples

Hereinafter, the present invention will be described by way of examples. However, the present invention is not limited to these examples.

(Example 1: Production of Food 1)

Tablets (about 200 mg per tablet) that contain an ethanol extract of pine bark (trade name: Flavangenol, produced by TOYO SHINYAKU Co., Ltd.) containing 40 wt% of proanthocyanidins (OPC content: 20 wt% in the extract) and 13 wt% of catechins, ascorbic acid (Maruzen Pharmaceuticals Co., Ltd.), crystalline cellulose, sucrose ester, silicon dioxide, and eggshell calcium in the amounts (parts by weight) shown in Table 1 below were produced. These tablets were referred to as "Food 1".

(Example 2: Production of Food 2)

Tablets (about 200 mg per tablet) that contain the same ethanol extract of pine bark (trade name: Flavangenol, produced by TOYO SHINYAKU Co., Ltd.) as in Example 1, crystalline cellulose, sucrose ester, silicon dioxide, and eggshell calcium in the weight ratios shown in Table 1 below were produced. These tablets were referred to as "Food 2".

(Example 3: Production of Food 3)

First, the same ethanol extract of pine bark (trade name: Flavangenol, produced by TOYO SHINYAKU Co., Ltd.) as in Example 1 was purified using Sephadex-LH20 under the conditions described below to prepare a pine bark extract containing 95.9 wt% of proanthocyanidins. Then, tablets (about 200 mg per tablet) containing this purified pine bark extract, crystalline cellulose, sucrose ester, silicon dioxide, and eggshell calcium in the weight ratios shown in Table 1 below were produced. These tablets were referred to as "Food 3".

(Purification of proanthocyanidins)

First, 100 mL of MCI Gel (manufactured by Mitsubishi Chemical Corporation) swollen with water were filled in a 30 × 300 mm column, and washed with 50 mL of purified water. Next, 400 mg of the above-described pine bark extract were dissolved in 8 mL of purified water, and this solution was applied on the above-described column so that proanthocyanidins were adsorbed. Then gradient elution was conducted using 0 to 30% (v/v) ethanol-water mixed solvents, and eluate was collected in fractions of 10 mL each. Each fraction was subjected to silica gel chromatography (TLC) to detect OPCs using a specimen of a dimeric OPC (dimer: proanthocyanidin B-2 (Rf value: 0.6)) as an indicator. The conditions of TLC were as follows:

    TLC: silica gel plate manufactured by Merck & Co., Inc.
    Eluent: benzene/ethyl formate/formic acid (2/7/1)
    Detection reagent: a mixture of sulfuric acid and anisaldehyde
    Sample amount: 10 μL each

The elution was immediately interrupted at the time when an OPC was detected by TLC, and then 1200 mL of ethanol were allowed to apply on the column to elute the adsorbed proanthocyanidins. This eluate and the fraction in which the OPCs were detected were combined, and the mixture was freeze-dried, and thus, 156 mg of dry powder was obtained. This operation was repeated, so that a predetermined amount of dry powder was obtained. In order to determine the presence or absence of catechins in this dry powder, TLC was performed using a specimen of catechin (Rf value: 0.8) as an indicator under the same conditions as described above. Catechins were not detected in the dry powder.

The proanthocyanidin content in this dry powder was measured using proanthocyanidin B-2 as a specimen according to the method by R. B. Broadhurst et al. (J. Sci. Fd. Agric., 1978, 29, sections 788-794), and was found to be 95.9 wt%.

(Example 4: Production of Food 4)

Tablets (about 200 mg per tablet) that contain a mixture of the same pine bark extract as in Example 1 and the pine bark extract used for Food 3 in Example 3 in a ratio of 4:6 (proanthocyanidin content: 73.1 wt%, catechin content: 5.2 wt%), crystalline cellulose, sucrose ester, silicon dioxide, and eggshell calcium in the weight ratios shown in Table 1 below were produced. These tablets were referred to as "Food 4".

(Example 5: Production of Food 5)

Tablets (about 200 mg per tablet) that contain a grape seed extract (proanthocyanidin content: 38 wt%, catechin content: 2 wt%, produced by KIKKOMAN CORPORATION), crystalline cellulose, sucrose ester, silicon dioxide, and eggshell calcium in the weight ratios shown in Table 1 below were produced. These tablets were referred to as "Food 5".

(Comparative Example 1: Production of Food 6)

Tablets (about 200 mg per tablet) that contain ascorbic acid (Maruzen Pharmaceuticals Co., Ltd.), crystalline cellulose, sucrose ester, silicon dioxide, and eggshell calcium in the weight ratios shown in Table 1 below were produced. These tablets were referred to as "Food 6".

Table 1

Unit: parts by weight

| Components | | Example Food 1 | Example Food 2 | Example Food 3 | Example Food 4 | Example Food 5 | Com.Ex. Food 6 |
|---|---|---|---|---|---|---|---|
| | | 1 | 2 | 3 | 4 | 5 | 1 |
| Components — Pine bark extract | Proanthocyanidins 40 wt% | 20 | 20 | – | – | – | – |
| | Catechins 13 wt% | | | | | | |
| | Proanthocyanidins 73.1 wt% | – | – | 20 | – | – | – |
| | Catechins 5.2 wt% | | | | | | |
| | Proanthocyanidins 95.9 wt% | – | – | – | 20 | – | – |
| | Catechins 0 wt% | | | | | | |
| Components — Grape seed extract | Proanthocyanidins 38 wt% | – | – | – | – | 20 | – |
| | Catechins 2 wt% | | | | | | |
| Additives | Ascorbic acid | 15 | – | – | – | – | 15 |
| | Crystalline cellulose | 10 | 10 | 10 | 10 | 10 | 10 |
| | Sucrose ester | 5 | 5 | 5 | 5 | 5 | 5 |
| | Silicon dioxide | 2 | 2 | 2 | 2 | 2 | 2 |
| | Eggshell calcium | 48 | 63 | 63 | 63 | 63 | 68 |

(Examples 6 to 10 and Comparative Example 2: Measurement of blood passage time of human blood and influence on blood platelet aggregation ability and blood components)

The blood passage time of human blood before and after ingestion of the above-described Foods 1 to 6 and the influence on the blood platelet aggregation ability and the blood components were examined in the following manner.

(Measurement of blood passage time of human blood)

A total of 36 healthy persons (18 males and 18 females) between the ages of 22 and 63 years served as experimental subjects, and the subjects were divided into six groups randomly except that the numbers of males and females were equal among the groups. Experimental subjects in one group ingested one tablet of Food 1 daily for two weeks. Similarly, the subjects in other groups ingested one tablet of Food 2, 3, 4, 5, or 6. One tablet of each of the Foods 1 to 4 contains 40 mg of the ethanol extract of pine bark. Blood samples were collected immediately before starting the ingestion of Foods 1 to 6 and one week and two weeks after the start of the ingestion. The blood samples were collected from the median cubital vein using a vacuum blood collection tube (manufactured by TERUMO CORPORATION: treated with heparin sodium) while the subjects were resting in a sitting position. The subjects did not have a breakfast on the days on which the blood samples were collected. The obtained blood (test blood) was immediately used for measurement of the blood passage time.

The blood passage time was measured using MC-FAN (manufactured by Hitachi Haramachi Electronics Co., Ltd.). As microfabricated channels serving as a blood vessel model through which blood flows, a silicon single crystal substrate (Bloody6-7; manufactured by Hitachi Haramachi Electronics Co., Ltd.) that is a substrate provided with a micro channel array having 8736 parallel micro grooves, each having a channel depth of 4.5 µm, a channel width (i. e., width of the channel at half depth of the channel) of 7 µm, and a channel length of 30 µm was used. Then, 100 µL of the blood were allowed to flow at a hydraulic pressure difference of 20 cm, and the passage time of the entire blood was measured as the blood passage time. Furthermore, the blood flow was filmed and recorded using a microscope-video camera system. For all measurement values, the average of the values obtained by three measurements was employed. The obtained blood passage time was corrected, taking the passage time required for 100 µL of physiological saline as 12 seconds. Table 2 shows the measurement results of the blood passage time. Each of the values in the table indicates the average value ± standard error of the flow rate in each group.

(Influence on blood platelet aggregation ability and blood components)

The blood platelet aggregation ability was evaluated after ingestion of each of the Food 1, 2, 3, 4, 5, and 6 in the following manner. Before the ingestion of Foods 1 to 6, two blood samples of 4.5 mL each were collected from the above-described subjects using two Venoject tubes (manufactured by TERUMO CORPORATION). The blood in one of the tubes was centrifuged at 1,000 rpm to form a supernatant in which no platelet aggregation occurred, and the supernatant was collected and poured into two cuvettes of 100 µL each. The blood in the other tube was centrifuged at 3,000 rpm so as to cause platelet aggregation, and a supernatant thereof was collected and poured into cuvettes of 100 µL each, which served as a blank. The two cuvettes containing the supernatant in which no platelet aggregation occurred and the blank were placed in an MCM Hema Tracer 313 (MC MEDICAL Co., LTD.), and the platelet aggregation rate was measured after the addition of 22 µL each of 30 µM ADP and 10 µM ADP to the supernatant in which no platelet aggregation occurred. The highest values in the groups were employed as the maximal platelet aggregation rate, and an average thereof was calculated. The calculated average value was 0.38% when 10 µM ADP was added, and 0.74% when 30 µM ADP was added. The same operation as described above was also performed after the two-week ingestion of the foods to measure the platelet aggregation rate in blood.

Based on the obtained platelet aggregation rate after the two-week ingestion of the foods, a relative value, where the average value of the maximal platelet aggregation rate before the ingestion was assumed to be 1, was calculated for each ADP concentration, and the relative values were averaged respectively. Table 3 shows the results.

In order to examine the influence on the blood components, blood samples were collected from the subjects before and after the two-week ingestion of the foods using Insepack-S (KYOKUTO PHARMACEUTICAL INDUSTRIAL CO., LTD.) and Insepack-E (KYOKUTO PHARMACEUTICAL INDUSTRIAL CO., LTD.), respectively (amount of collected blood sample was 9 mL and 2 mL, respectively). These blood samples were entrusted to Kurume Rinsho-Kensa Center (Kurume Clinical Laboratory Center) to determine the contents of protein and lipid, erythrocyte count, leukocyte count, platelet count, hematocrit value, and amount of glucose in blood. Relative values of each of the values after the ingestion of the foods with respect to the corresponding value before the ingestion of the foods were calculated and averaged. Table 3 shows the results.

Table 2

|  | Ex.6 | Ex.7 | Ex.8 | Ex.9 | Ex.10 | Com.Ex.3 |
|---|---|---|---|---|---|---|
| Ingested food | Food 1 | Food 2 | Food 3 | Food 4 | Food 5 | Food 6 |
| Before ingestion | 50.2±1.3 | 50.0±0.9 | 50.1±3.1 | 50.6±1.1 | 50.2±2.1 | 49.7±2.0 |
| After one-week ingestion | 41.1±1.4 | 43.9±2.0 | 45.9±2.2 | 44.6±2.1 | 48.9±1.2 | 49.0±1.7 |
| After two-week ingestion | 40.8±1.2 | 42.6±1.8 | 44.4±1.7 | 42.9±1.4 | 46.8±1.3 | 49.5±1.6 |
| Average value ± standard error (unit: second) | | | | | | |

Table 3

| | | Ex.6 | Ex.7 | Ex.8 | Ex.9 | Ex.10 | Com.Ex.3 |
|---|---|---|---|---|---|---|---|
| Ingested food | | Food 1 | Food 2 | Food 3 | Food 4 | Food 5 | Food 6 |
| Platelet aggregation ability | 10μM ADP | 1.05±0.05 | 0.97±0.06 | 1.02±0.04 | 1.03±0.04 | 1.06±0.03 | 1.03±0.05 |
| | 30μM ADP | 1.06±0.03 | 1.00±0.03 | 1.06±0.05 | 1.01±0.05 | 1.01±0.03 | 1.04±0.04 |
| Protein | Total protein | 0.99±0.02 | 0.99±0.03 | 1.02±0.04 | 1.02±0.07 | 1.02±0.05 | 0.99±0.03 |
| | A/G ratio | 0.97±0.06 | 1.01±0.05 | 0.98±0.04 | 1.04±0.04 | 1.03±0.04 | 1.01±0.07 |
| Lipid | Total cholesterol | 1.01±0.06 | 1.01±0.10 | 1.03±0.05 | 0.99±0.09 | 1.00±0.08 | 1.03±0.07 |
| | Neutral fat | 1.02±0.20 | 1.04±0.44 | 0.99±0.18 | 1.03±0.23 | 0.98±0.28 | 1.04±0.26 |
| | Free fatty acid | 0.99±0.17 | 1.06±0.24 | 1.01±0.20 | 1.02±0.20 | 1.04±0.19 | 0.96±0.17 |
| | Phospholipid | 1.01±0.12 | 0.99±0.05 | 1.06±0.11 | 1.03±0.14 | 1.01±0.06 | 0.93±0.10 |
| Erythrocyte count | | 1.02±0.03 | 0.96±0.03 | 1.00±0.06 | 1.05±0.05 | 0.98±0.05 | 0.99±0.02 |
| Leukocyte count | | 1.02±0.15 | 0.95±0.14 | 1.04±0.07 | 1.01±0.08 | 1.00±0.09 | 0.96±0.12 |
| Platelet count | | 1.01±0.11 | 0.98±0.05 | 1.02±0.05 | 0.98±0.04 | 1.00±0.06 | 0.99±0.08 |
| Hematocrit value | | 1.01±0.03 | 0.97±0.03 | 0.99±0.03 | 1.03±0.02 | 0.98±0.05 | 0.99±0.02 |
| Amount of blood glucose | | 0.98±0.10 | 1.05±0.15 | 1.03±0.20 | 1.02±0.17 | 1.01±0.15 | 0.98±0.11 |
| Average value ± standard error | | | | | | | |

It can be seen from the results in Table 2 that the blood passage time was significantly decreased and the fluidity of blood was improved by ingesting any one of the Foods 1 to 5 produced according to the compositions of the present invention. Moreover, the longer the blood passage time of the subject before the ingestion is, the greater the effect of improving the fluidity of blood tended to be. The state of blood flow was observed before and after the ingestion of the foods using MC-FAN to find no clogging in the micro channel array in both cases. Furthermore, when comparing Food 2 with Food 5 both having the same amount of proanthocyanidins, it can be seen that the pine bark extract provides a higher effect of improving the fluidity of blood than the grape seed extract do. Comparison of Foods 2 to 4 shows that among the plant extracts, the plant extracts containing proanthocyanidins in a ratio of less than 75 wt% exhibited a higher activity. Moreover, Food 1 containing proanthocyanidins and ascorbic acid had the best blood fluidity improvement effect.

The results in Table 3 show that platelet aggregation, platelet count, kinds and contents of blood plasma components such as protein and lipid, blood cell count such as erythrocyte count and leukocyte count, and hematocrit value that is a volume ratio of blood cells per blood before and after the ingestion of Foods 1 to 5, all of which are related to the fluidity of blood, were not changed from those before the ingestion. This indicates that the effect of improving the fluidity of blood of Foods 1 to 5 was achieved not by an influence on the components in blood plasma and on the blood platelets or a change in erythrocyte count and leukocyte count, but by an improvement of the fluidity of erythrocytes and leukocytes.

The foregoing shows that foods containing proanthocyanidins provide the effect of improving the fluidity of blood associated with improvement of the blood cell fluidity. Furthermore, it can be recognized that among plant extracts, pine bark extracts provide the above-mentioned excellent effects of proanthocyanidins, and pine bark extracts containing proanthocyanidins in a ratio of less than 80 wt% provide particularly excellent effects.

(Examples 11 to 15 and Comparative Example 3: Evaluation of blood fluidity improvement effect)

The blood fluidity improvement effect was evaluated in the following manner. First, the blood flow rate of the six subjects in each group described above was measured before ingestion. Then, the subjects in one group ingested one tablet of Food 1 daily for two weeks. Similarly, the subjects in other groups ingested one tablet of Food 2, 3, 4, 5, or 6. The blood flow rate was measured again after the two-week ingestion. The blood flow rate was measured at a region under the right forearm skin using a rheometer (laser blood perfusion imager PIM II; Perimed AB, Sweden). Table 4 shows the results. Each of the values in the table indicates the average value ± standard error, and larger values indicate a higher blood flow rate.

Table 4

|  | Food | Before ingestion | After ingestion | After ingestion - Before ingestion |
|---|---|---|---|---|
| Ex.11 | Food 1 | 1.33±0.04 | 1.60±0.04 | 0.27±0.03 |
| Ex.12 | Food 2 | 1.34±0.05 | 1.57±0.04 | 0.23±0.03 |
| Ex.13 | Food 3 | 1.34±0.03 | 1.41±0.03 | 0.07±0.03 |
| Ex.14 | Food 4 | 1.36±0.05 | 1.55±0.07 | 0.19±0.05 |
| Ex.15 | Food 5 | 1.27±0.03 | 1.40±0.03 | 0.13±0.02 |
| Com.Ex.3 | Food 6 | 1.35±0.03 | 1.34±0.05 | -0.01±0.04 |
| Average value ± standard error | | | | |

Referring to Table 4, since the blood flow rate was increased in the groups ingesting Foods 1 to 5, it can be recognized that the compositions for improving blood fluidity of the present invention achieved an increase in the blood flow rate in tissue, that is, the blood fluidity improvement effect was obtained. In particular, the pine bark extracts exhibited a higher blood fluidity improvement effect than the grape seed extract having almost the same proanthocyanidin content. Furthermore, as in the case of the blood passage time evaluation, the pine bark extract containing proanthocyanidins in a ratio of about 73 wt% exhibited a higher blood fluidity improvement effect than the pine bark extract containing proanthocyanidins in a ratio of about 96 wt%. Moreover, Food 1 containing proanthocyanidins and ascorbic acid provided the best blood fluidity improvement effect.

(Examples 16 to 18 and Comparative Example 4: Cold water load test)

In order to examine the effect of ingestion of Foods 1, 2, 5, or 6 on peripheral blood vessels, a cold water load test described later was performed, in which peripheral capillary vessels were constricted so as to cause temporary blood circulation disorder, and then the effect of recovering blood flow was determined.

First, 15 healthy male subjects between the ages of 20 and 53 years were randomly divided into three groups. Before ingestion of the foods, a cold water load test described below was performed. The subjects in one group ingested one tablet of Food 1 daily for one week. Similarly, the subjects in other groups ingested one tablet of Food 2, 5, or 6. The cold water load test was further performed after the one-week ingestion. The cold water load test described above was performed in the following manner. The subjects soaked their left hand in cold water at 15°C for 10 seconds, and skin temperature was measured immediately after and 10 minutes after the cold water load using thermography (TVS 600, Nippon Avionics Co., Ltd.). Then, an average temperature of skin temperatures at three positions, i.e., the tip of the middle finger, the midpoint of the proximal phalanx of the middle finger, and the midpoint of the third metacarpal bone, was determined as an average skin temperature at the opisthenar. Table 5 shows the results.

Table 5

| Ingested food | Cold water load | Skin temperature(°C)*1 | | Recovery of skin temperature (°C)*2 | | Recovery of skin temperature before and after ingestion of food (°C) |
|---|---|---|---|---|---|---|
| | | Before ingestion | After ingestion | Before ingestion | After ingestion | |
| Ex.16 Food 1 | Immediately after | 23.67±0.77 | 24.01±0.54 | 2.79 | 7.11 | 4.32 |
| | After 10 min. | 26.46±1.10 | 31.12±1.29 | | | |
| Ex.17 Food 2 | Immediately after | 23.82±0.39 | 23.89±0.49 | 2.96 | 4.29 | 1.33 |
| | After 10 min. | 26.78±1.16 | 28.18±0.98 | | | |
| Ex.18 Food 5 | Immediately after | 24.10±0.81 | 24.01±0.54 | 2.02 | 2.97 | 0.95 |
| | After 10 min. | 26.12±1.02 | 26.98±1.13 | | | |
| Com.Ex.4 Food 6 | Immediately after | 23.89±0.77 | 24.11±0.99 | 3.00 | 3.30 | 0.30 |
| | After 10 min. | 26.89±1.21 | 27.41±1.19 | | | |

*1: Average value ± standard error

*2: (Temperature after 10 minutes after the cold water load) − (Temperature immediately after the cold water load)

As can be seen from Table 5, in the groups ingesting Foods 1, 2, or 5 produced according to the compositions of the present invention, the temperature of the skin was increased more than in the group ingesting Food 6. This shows that the compositions of the present invention provided the effect of improving blood fluidity, and also strengthened blood vessels. Furthermore, the capillary vessels quickly recovered from the constricted state to the normal state.

In particular, Food 1 containing proanthocyanidins and ascorbic acid had the best blood fluidity improvement effect.

(Example 19: Production of tea drink containing proanthocyanidins 1)

First, 1 L of water at 55°C was added to 12 g of green tea, and extraction was performed for 4 minutes, and then the tea leaves were removed by centrifugation to obtain an extract liquid. A pine bark extract was dissolved in this extract liquid so that the amount of proanthocyanidins was 200 mg/L. After 20 mg/L vitamin C was further added to the resultant solution, pH was adjusted to 6.0 using sodium bicarbonate, and thus a drink was obtained.

(Comparative Example 5)

A drink was prepared in the same manner as in Example 15 except that no pine bark extract was added.

(Example 20: Examination of blood fluidity improvement effect of tea drink containing proanthocyanidins)

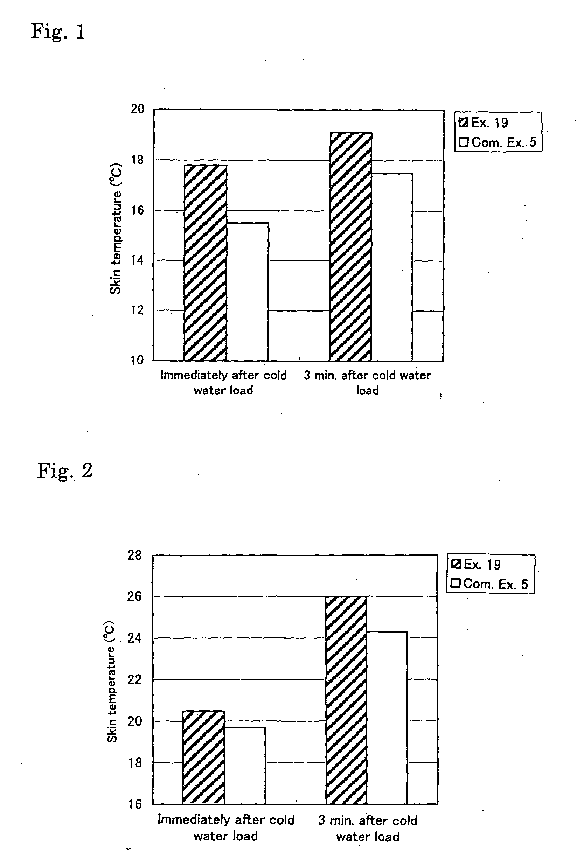

In order to confirm the blood fluidity improvement effect of the proanthocyanidin-containing drink of Example 19, a cold water load test was performed on two females having poor circulation in the following manner. First, the subjects were prohibited from eating and drinking for three hours until ingestion of the drink. Then, the subjects ingested 200 mL of the drink of Comparative Example 5. Thirty minutes after the ingestion, the subjects soaked their left hand in water at 10°C for 30 seconds, and an increase in fingertip temperature was measured immediately after and 3 minutes after the cold water load using thermography (TVS 600, Nippon Avionics Co., Ltd.). The fingertip temperature was measured in the same manner as in Example 16. On the next day, the subjects ingested the proanthocyanidin-containing drink of Example 19 in the same manner as in the case where they had ingested the above-described drink of Comparative Example 5, and the fingertip temperature was measured to examine the effect of improving peripheral blood fluidity. It should be noted that when the fingertip temperatures were measured using thermography before the ingestion of the drink of the comparative example and also before the ingestion of the drink of the example, there was no difference in the temperatures. Figs. 1 and 2 show the results.

The results in Figs. 1 and 2 show that when the subjects ingested 200 mL of the proanthocyanidin-containing drink of Example 19, both the fingertip temperatures immediately after and 3 minutes after the cold water load after the ingestion were higher than in the case where they ingested the drink of Comparative Example 5 that did not contain proanthocyanidins. From this result, it was confirmed that also when proanthocyanidins were contained in a drink, the blood fluidity improvement effect was achieved by ingesting such proantyochyanidin-containing drink, and it was found that the effect can be achieved more quickly by ingestion in the form of a drink than in other forms. Moreover, although not shown in the data, the proanthocyanidin-containing tea drink of Example 19 provided a higher blood fluidity improvement effect than other drinks containing the same amount of proanthocyanidins.

(Example 21: Production of tea drink containing proanthocyanidins 2)

First, 1 L of water at 80°C was added to 24 g of green tea leaves, and extraction was performed for 5 minutes, and then the tea leaves were removed by filtration to obtain an extraction liquid. This extraction liquid was diluted three-fold with water to prepare a tea drink. Then, a pine bark extract (containing 40 wt% of proanthocyanidins) and ascorbic acid were added to this tea drink in the amounts shown in Table 6 below, and thus a drink was produced in an amount of 200 mL.

(Example 22: Production of tea drink containing proanthocyanidins 3)

A drink was produced in the same manner as in Example 21 except that the pine bark extract and ascorbic acid were added in the amounts shown in Table 6 below.

(Comparative Example 6)

A drink was produced in the same manner as in Example 21 with an exception that the pine bark extract and ascorbic acid were added in the amounts shown in Table 6 below.

Table 6

|  | Ex.21 | Ex.22 | Com.Ex.6 | Control example |
|---|---|---|---|---|
| Pine bark extract | 40 | 40 | - | - |
| Ascorbic acid | 100 | - | 100 | - |
| *Content (mg) per 200 mL drink | | | | |

(Example 23: Evaluation of blood fluidity improvement effect and effect of recovering blood flow of tea drink containing proanthocyanidins)

In order to examine the blood fluidity improvement effect of the proanthocyanidin-containing tea drinks in more detail, the blood fluidity improvement effect in a single ingestion was evaluated using the same rheometer as in Examples 11 to 15. First, seven subjects were gathered in the morning fasting, and after the subjects rested for one hour, the blood flow rate in right hand forefinger was measured. This was determined as blood flow rate before ingestion. Then, the subjects ingested the drink of Example 21. One hour after the ingestion of the drink, the blood flow rate was again measured, and this was determined as blood flow rate after ingestion. Regarding subjects in a control group, the blood flow rate was previously measured before and after ingestion of a tea drink containing neither a pine bark extract containing proanthocyanidins nor ascorbic acid, and the blood flow rate before ingestion and the blood flow rate after ingestion were measured. Based on these measurement values, the rate of increase of blood flow relative to the control group was obtained by correcting an error between the tests using the following formula.

$$\text{Rate of increase in blood flow (\%)} = (A - B) \times 100$$

$$A = \frac{\left(\begin{array}{c}\text{Blood flow rate after ingestion}\\\text{in test group}\end{array}\right) - \left(\begin{array}{c}\text{Blood flow rate after ingestion}\\\text{in control group}\end{array}\right)}{\left(\begin{array}{c}\text{Blood flow rate after ingestion}\\\text{in control group}\end{array}\right)}$$

$$B = \frac{\left(\begin{array}{c}\text{Blood flow rate before ingestion}\\\text{in test group}\end{array}\right) - \left(\begin{array}{c}\text{Blood flow rate before ingestion}\\\text{in control group}\end{array}\right)}{\left(\begin{array}{c}\text{Blood flow rate before ingestion}\\\text{in control group}\end{array}\right)}$$

Furthermore, in order to evaluate the effect of recovering blood flow, that is, in order to comprehensively evaluate the effects of improving the elasticity and flexibility of blood vessels and the effects of improving the fluidity of blood cells and fluidity of blood, a cold water load test was performed in which the subjects soaked their right hand in ice water for 10 seconds, and the blood flow rate was measured at predetermined times immediately after cold water load (1 minute, 2 minutes, and 5 minutes after the cold water load), and the rate of increase in blood flow was obtained according to the same formula as described above. In more detail, the blood flow rate after the cold water load was employed in place of the blood flow rate after ingestion in the test group in the above formula. Also, the cold water load test was performed on the control group after the ingestion. In the control group, the blood flow rate at the corresponding predetermined times after the cold water load was employed as the blood flow rate after ingestion in the control group in the above formula. Table 7 shows the results. The greater values in Table 7 indicate that the blood flow rate was highly increased compared with the case in which the drink of the control example was ingested.

Regarding the drinks of Example 22 and Comparative Example 6, the same subjects as described above ingested the drinks, and the same tests were performed to measure the rate of increase in blood flow. The interval between the tests (i.e., an interval from a measurement of the rate of increase in blood flow to the next measurement) was at least two hours so that the measurement was not affected by the former ingestion of drink. Table 7 shows the results.

Table 7

| | Rate of increase in blood flow | | | |
|---|---|---|---|---|
| | One hour after ingestion | Cold water load test | | |
| | | After 1 min. | After 2 min. | After 5 min. |
| Ex.21 | 22.6 | 15.9 | 22.8 | 24.6 |
| Ex.22 | 2.1 | 1 | 9.6 | 4.4 |
| Com.Ex.6 | -0.8 | -8.1 | -11.2 | -4.7 |
| Unit: % | | | | |

The results in Table 7 show that in the case of the tea drink containing the pine bark extract that contains proanthocyanidins of the present invention or tea drink containing the pine bark extract and ascorbic acid (Examples 21 and 22), the blood flow rate was more increased than in the case of the tea drink containing neither the pine bark extract containing proanthocyanidins nor ascorbic acid (control example). Furthermore, such an increase in the blood flow rate was not observed in the case of the tea drink containing only ascorbic acid (Comparative Example 6), so that it is found that the blood fluidity improvement effect was promoted by a single administration of the drink containing proanthocyanidins. Furthermore, the drinks of the examples (Examples 21 and 22) achieved a larger amount of increase in the blood flow rate at the predetermined times after the cold water load than the drink containing only ascorbic acid (Comparative Example 6) and the tea drink containing neither a pine bark extract nor ascorbic acid (control example) did. In this way, it was found that the blood flow rate recovered after the cold water load in Examples 21 and 22. This may be caused by the recovery of the constricted blood vessels and the inflow of blood, and therefore, it is considered that the flexibility and elasticity of blood vessels are improved, and the fluidity of blood cells and the fluidity of blood, are improved. In particular, the drink containing ascorbic acid and the pine bark extract containing proanthocyanidins (Example 21) showed considerably higher effects of increasing the blood flow rate and recovering blood flow than the drink containing only the pine bark extract (Example 22), so that it was also found that a synergistic effect was achieved by the combination of proanthocyanidins and ascorbic acid together.

Separately, the same test was performed by the use of a drink containing a pine bark extract and ascorbic acid in purified water instead of a tea drink. The rate of increase in blood flow one hour after the ingestion was found to be 15.1%, so that it was also found that Example 21, which employed the form of a tea drink, has higher effects.

(Example 24: Sensory evaluation of tea drink containing proanthodyanidins)

Various types of tea drinks, namely green tea, pine needle tea, oolong tea, black tea, and barley tea, were added with proanthocyanidins, and the palatability of the resultant various types of tea drinks containing proanthocyanidins were evaluated in the following manner. First, 1 L of water at 80°C was added to 10 g of tea leaves (green tea, pine needle, oolong tea, and black tea), and extraction was performed for 5 minutes, and then the tea leaves were removed by filtration to obtain tea drinks. Separately, 30 g of barley were soaked in 1 L of water, and boiled for 10 minutes to obtain a barley tea drink. A pine bark extract (containing 40 wt% of proanthocyanidins) was added to each tea drink in such a ratio that it is contained in an amount of 40 mg per 350 mL tea drink and stirred, and thus tea drinks containing proanthocyanidins were prepared. Six persons (subjects) tasted these tea drinks containing proanthocyanidins and ranked them in order of preference, and the ranking was calculated into scores using the evaluation criteria listed below. Table 8 shows the results:

(Evaluation criteria)

| | |
|---|---|
| First preference | 5 points |
| Second preference | 4 points |
| Third preference | 3 points |

(continued)

| Fourth preference | 2 points |
|---|---|
| Fifth preference | 1 point |

Table 8

| | Subject | | | | | | Total score |
|---|---|---|---|---|---|---|---|
| | a | b | c | d | e | f | |
| Green tea | 5 | 5 | 4 | 4 | 5 | 5 | 28 |
| Pine needle tea | 4 | 2 | 5 | 5 | 4 | 1 | 21 |
| Oolong tea | 3 | 3 | 2 | 1 | 2 | 3 | 14 |
| Black tea | 2 | 4 | 3 | 2 | 3 | 4 | 18 |
| Barley tea | 1 | 1 | 1 | 3 | 1 | 2 | 9 |
| The numbers indicate the scores. | | | | | | | |

From the results in Table 8, it can be recognized that the tea drinks (green tea, pine needle tea, oolong tea, and black tea) that were prepared from plant leaves have better palatability than the tea drink (barley tea) that was prepared from grain has. In particular, regarding the teas that were prepared from plant leaves, there were remarks as follows: "The astringent taste derived from proanthocyanidins or pine bark was decreased," or "The flavor was improved." Among these teas, it was found that especially green tea has excellent palatability.

(Example 25: Production of tea drink containing a high concentration of proanthocyanidins)

A drink was prepared by adding proanthocyanidins into green tea (roasted tea). In more detail, 1 L of water at 85 °C were added to 7 g of roasted tea, and extraction was performed for 4 minutes. Then, the tea leaves were removed by centrifugation to obtain an extract liquid. A pine bark extract containing 40 wt% of proanthocyanidins (OPC content: 20 wt% in proanthocyanidins) was dissolved in this extraction liquid so that it was contained in a ratio of 100 mg/L. After 800 mg/L vitamin C was further added to this solution, pH was adjusted to 6.0 using sodium bicarbonate, and thus a drink was obtained.

(Example 26: Production of drink containing a low concentration of proanthocyanidins)

As a soft drink, a drink containing a pine bark extract in a ratio of 10 mg/L was prepared. The components and the amounts of the drink are listed below.

| Components | Amount (weight per 1 L) |
|---|---|
| Fructose-glucose syrup | 110 g |
| Citric acid | 0.2 g |
| L-Ascorbic acid | 0.2 g |
| Pine bark extract | 10 mg |
| Calcium chloride | 840 mg |
| Magnesium chloride | 80 mg |
| Potassium chloride | 280 mg |
| Flavor | 0.15 g |
| Purified water | sufficient quantity |

(Example 27: Production of drink containing a high concentration of proanthocyanidins)

As a fruit juice drink, a drink in which a pine bark extract containing 40 wt% of proanthocyanidins (OPC content: 20 wt% in proanthocyanidins) was contained in a ratio of 200 mg/L was prepared. The components mixed and the mixing amounts are listed below.

| Components | Amount (weight per 1 L) |
|---|---|
| Fructose-glucose syrup | 40 g |

| Lemon juice | 30 g |
|---|---|
| Citric acid | 2 g |
| L-Ascorbic acid | 10 g |
| Pine bark extract | 200 mg |
| Flavor | 0.15 g |
| Vitamin B6 | 1 mg |
| Vitamin B12 | 2 mg |
| Purified water | sufficient quantity |

(Examples 28 and 29 and Comparative Example 7: Examination of blood vessel protection properties)

In order to examine the blood vessel protection properties of proanthocyanidins, which are active components of the composition of the present invention, the effect on the elasticity of rat blood vessels was evaluated. First, male SHR rats at the age of four weeks (Disease Model Cooperative Research Association) were given a standard feed (MF powder: Oriental Yeast Co., Ltd.) and water for one week for acclimation, and then divided into groups, each including 5 rats such that the average of weight was almost equal among the groups. Then, the rats were allowed to freely ingest a feed that is the standard feed containing an ethanol extract of pine bark (trade name: Flavangenol, produced by TOYO SHINYAKU Co., Ltd.) in a ratio of 0.5 wt% (Feed 1) or 2.5 wt% (Feed 2), or a feed that is only the standard feed (Feed 3) for 28 days. Also, the rats in all of the groups were allowed to freely ingest a drinking water containing 1% of NaCl from the day when feeding started. On the day 28, thoracic aorta was excised to evaluate physical properties. A tension tester (EZ-test, SHIMADZU CORPORATION) was used for the measurement, and the thoracic aorta was stretched at a crosshead speed of 2 mm/min until it was ruptured. Thus, a stress-variation curve was obtained, whereby modulus of elasticity (gradient obtained from the stress-variation curve using the least-squares method) was calculated.
Table 9 shows the measurement results of the modulus of elasticity. The lower the modulus of elasticity is, the higher the elasticity of blood vessels is.

Table 9

| | Ethanol extract of pine bark (wt%) | Modulus of elasticity (N/mm$^2$) (Average value ± standard error) |
|---|---|---|
| Ex.28 | 0.50 | 4.02±0.53 |
| Ex.29 | 0.25 | 3.78±0.40 |
| Com.Ex.7 | - | 4.61±0.44 |

Table 9 shows that in both of the groups of Feeds 1 and 2 that contain proanthocyanidins, the elasticity of blood vessels was more improved than in the group of only the standard feed. From the above results, it was found that proanthocyanidins had the blood vessel protection properties.

Industrial Applicability

As described above, by ingesting a composition containing proanthocyanidins as active components, not only an excellent effect of improving blood fluidity but also an effect of protecting blood vessels can be achieved. When ingesting a composition further containing ascorbic acid, better effects can be achieved. In particular, this composition is useful as a beverage (beverage composition) containing proanthocyanidins and ascorbic acid or a derivative thereof.

**Claims**

1. A composition for improving blood fluidity, comprising a proanthocyanidin as an active component.

2. The composition of claim 1, wherein the composition improves blood cell fluidity.

3. The composition for improving blood fluidity of claim 1 or 2, further comprising ascorbic acid or a derivative thereof.

4. The composition for improving blood fluidity of any one of claims 1 to 3, wherein the proanthocyanidin comprises at least 20 wt% of OPC (oligomeric proanthocyanidin).

5. A beverage comprising a proanthocyanidin and ascorbic acid or a derivative thereof.

6. The beverage of claim 5, wherein the proanthocyanidin and the ascorbic acid or derivative thereof are contained at a weight ratio of 1 : 0.1 to 1:500.

7. The beverage of claim 5 or 6, wherein the proanthocyanidin is contained in the beverage in a concentration of 1 mg/L or more.

8. The beverage of any one of claims 5 to 7, wherein the beverage is a tea drink.

9. The beverage of any one of claims 5 to 8, wherein the beverage has blood fluidity improvement properties.

Fig. 1

Fig. 2

EP 1 547 591 A1

**INTERNATIONAL SEARCH REPORT**

| International application No. |
|---|
| PCT/JP03/09617 |

**A. CLASSIFICATION OF SUBJECT MATTER**
Int.Cl$^7$ A61K31/352, 31/375, A61P9/00, 43/00, C07D311/62, A23L1/30, 1/302, 2/00, A23F3/14

According to International Patent Classification (IPC) or to both national classification and IPC

**B. FIELDS SEARCHED**

Minimum documentation searched (classification system followed by classification symbols)
Int.Cl$^7$ A61K31/352, 31/375, A61P9/00, 43/00, C07D311/62, A23L1/30, 1/302, 2/00, A23F3/14

Documentation searched other than minimum documentation to the extent that such documents are included in the fields searched

Electronic data base consulted during the international search (name of data base and, where practicable, search terms used)
CAPLUS(STN), CAOLD(STN), REGISTRY(STN), JICST(JOIS)

**C. DOCUMENTS CONSIDERED TO BE RELEVANT**

| Category* | Citation of document, with indication, where appropriate, of the relevant passages | Relevant to claim No. |
|---|---|---|
| X<br>Y | EP 694305 A1 (INDENA S.p.A.),<br>31 January, 1996 (31.01.96),<br>Full text<br>& JP 8-59463 A<br>Full text<br>& AU 9527180 A        & US 5665365 A | 1,2,4<br>3 |
| X<br>Y | Satoshi TAKAGAKI, "Icho Ha Extract no Tokusei to Riyo", New Food Industry, 1998, Vol.40, No.5, pages 1 to 7 | 1,2<br>3 |

☒ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 October, 2003 (16.10.03) | 11 November, 2003 (11.11.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

22

## INTERNATIONAL SEARCH REPORT

| | International application No. |
|---|---|
| | PCT/JP03/09617 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | EP 1013278 A1 (JI, Ling),<br>28 June, 2000 (28.06.00),<br>Full text<br>& JP 2001-508777 A<br>Full text<br>& AU 9715884 A          & CN 1245433 A<br>& KR 2001049156 A     & US 6329000 B1<br>& WO 98/32455 A1 | 1,2<br>3 |
| X<br>Y | EP 1086693 A1 (KYOWA HAKKO KOGYO CO., LTD.),<br>28 March, 2001 (28.03.01),<br>Full text<br>& JP 2001-158739 A<br>Full text<br>& AU 200059478 A          \ & CN 1288688 A<br>& KR 2001076199 A | 5-7,9<br>3 |
| X<br>Y | EP 1135995 A2 (KYOWA HAKKO KOGYO CO., LTD.),<br>26 September, 2001 (26.09.01),<br>Full text<br>& JP 2001-270881 A<br>Full text<br>& AU 200129820 A          & CN 1321444 A<br>& KR 2001100837 A     & US 2001/036487 A1 | 5-7,9<br>3 |
| X | EP 713706 A2 (SUNTORY LTD.),<br>29 May, 1996 (29.05.96),<br>Full text; particularly, example 9<br>JP 8-225453 A<br>Full text; particularly, example 9<br>& US 5607965 A          & FI 9505691 A<br>& NO 9504834 A | 5-9 |
| X | JP 2002-29953 A (Sunstar Inc.),<br>29 January, 2002 (29.01.02),<br>Full text; particularly, example 8<br>(Family: none) | 5-7,9 |
| X | EP 815857 A1 (SUNTORY LTD.),<br>07 January, 1998 (07.01.98),<br>Full text; particularly, example 7<br>& JP 9-291039 A<br>Full text; particularly, example 7<br>& US 6294190 B1          & WO 97/23210 A1<br>& KR 98702533 A | 5-7,9 |
| A | SHUMEJKO, Pavel et al., THE EFFECT OF SIMULATED<br>ACID RAIN ON THE BIOCHEMICAL COMPOSITION OF<br>SCOTS PINE (PINUS SYLVESTRISL.), NEEDLES,<br>Environmental Pollution, 1996, Vol.92, No.3,<br>pages 315 to 321 | 1-9 |

Form PCT/ISA/210 (continuation of second sheet) (July 1998)

## INTERNATIONAL SEARCH REPORT

International application No.

PCT/JP03/09617

C (Continuation).   DOCUMENTS CONSIDERED TO BE RELEVANT

| Category* | Citation of document, with indication, where appropriate, of the relevant passages | Relevant to claim No. |
|---|---|---|
| A | NYMAN, Bengt F., PROTEIN-PROANTHOCYANIDIN INTERACTIONS DURING EXTRACTION OF SCOTS PINE NEEDLES, Phytochemistry, 1985, Vol.24, No.12, pages 2939 to 2944 | 1-9 |
| A | SANTOS-BUELGA, CELESTINO et al., Proanthocyanidins and tannin-like compounds-nature, occurrence, cietary intake and effects on nutrition and health, J.Sci.Food Agric., 2000, Vol.80, pages 1094 to 1117 | 1-9 |
| A | EP 1208755 A1 (Meiji Seika Kaisha, Ltd.), 29 May, 2002 (29.05.02), Full text & WO 01/001798 A1      & AU 200057068 A & KR 2002019481 A | 1-9 |
| P,X | JP 2003-95968 A (Nobuyoshi TANAKA), 03 April, 2003 (03.04.03), Full text (Family: none) | 1-3 |
| P,X | JP 2003-128560 A (Kikkoman Corp.), 08 May, 2003 (08.05.03), Full text (Family: none) | 1,2 |
| P,X | JP 2003-284525 A (Kabushiki Kaisha Hayashibara Seibutsu Kagaku Kenkyujo), 07 October, 2003 (07.10.03), Full text; particularly, example 4 (Family: none) | 5-7,9 |

Form PCT/ISA/210 (continuation of second sheet) (July 1998)

# INTERNATIONAL SEARCH REPORT

| International application No. |
| --- |
| PCT/JP03/09617 |

**Box I    Observations where certain claims were found unsearchable (Continuation of item 2 of first sheet)**

This international search report has not been established in respect of certain claims under Article 17(2)(a) for the following reasons:

1. ☐ Claims Nos.:

because they relate to subject matter not required to be searched by this Authority, namely:

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

Claims 1 to 4 and 9 relate to a composition improving the fluidity of blood which contains proanthocyanidine as the active ingredient.
Claims 5 to 8 relate to a drink which contains proanthocyanidine and ascorbic acid or its derivative.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (1)) (July 1998)